# FASCICULE DE BREVET EUROPEEN

(11) **EP 0 848 002 B1**
(45) Date de publication et mention de la délivrance du brevet: **10.10.2001**
(21) Numéro de dépôt: 97402942.3
(22) Date de dépôt: 05.12.1997
(51) Int. Cl.: C07D 405/12, C08F 8/46

(54) **Ester de l'acide maléimidobenzoique**
Maleimidobenzosäure Ester
Ester of maleimidobenzoic acid

(30) Priorité: 12.12.1996 FR 9615472
(43) Date de publication de la demande: 17.06.1998
(73) Titulaire: INSTITUT FRANCAIS DU PETROLE, 92502 Rueil-Malmaison (FR)
(72) Inventeur: Garapon, Jacques, 69003 Lyon (FR); Vallet, Jacques, 69008 Lyon (FR)

(56) Documents cités:
- DE-A- 2 626 769
- US-A- 4 189 560

## Description

La présente invention concerne des nouveaux composés chimiques bifonctionnels, leurs préparations et leurs utilisations.

Dans le cadre d'une recherche sur les alliages de polymères comprenant au moins une polyoléfine et au moins un polymère incompatible, la demanderesse a été amenée à s'intéresser aux composés polyfonctionnels comportant, d'une part, une fonction susceptible de se greffer facilement sur une chaîne paraffinique par voie radicalaire et, d'autre part, une fonction susceptible de former une liaison par voie ionique avec des polymères comme par exemple les polybutylène-téréphtalates ou les polyamides.

C'est ainsi que la demanderesse a synthétisé des composés comportant une double liaison maléimide capable de former radicalairement, en présence ou en absence d'un initiateur radicalaire, une liaison avec une chaîne paraffinique, et un groupement époxyde capable de réagir sur des groupes fonctionnels, par exemple des groupes terminaux de certains polymères.

Les composés de la présente invention répondent à la formule B ci-après dans laquelle n est zéro ou un nombre entier de 1 à 4, bornes incluses, X est un un reste hydrocarboné ou un atome d'halogène, les groupes X étant identiques ou différents lorsque n est un nombre de 2 à 4, et R est un groupe divalent choisi parmi les groupes hydrocarbonés substitués ou non substitués, de préférence non sustitués.

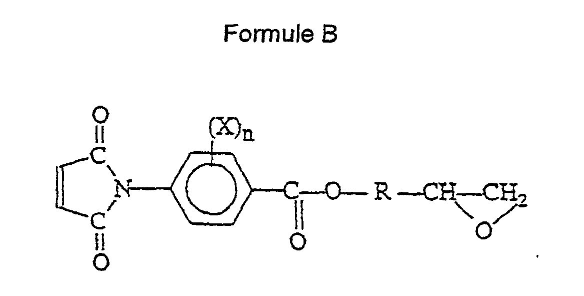

Dans le cas le plus simple, n est égal à zéro et les esters formés sont ceux de l'acide maléimidobenzoïque et R est un reste méthylène.

La synthèse de ces esters de l'acide 4-maléimidobenzoïque substitué ou non substitué sur le cycle benzénique de formule A ci-après a été entreprise pour bénéficier de la bonne aptitude de la fonction maléimide à se greffer sur une paraffine sans initiateur peroxyde. Ce greffage radicalaire sur une polyoléfine est généralement effectué avec du méthacrylate de glycidyle ou d'autres dérivés insaturés comme cela est par exemple décrit dans l'article de C.M. CHEN et coll. publié dans Annu. Tech. Conf. Soc. Plast. Eng. (52nd) 2002, 1994. Le rôle de la fonction époxyde est de réagir par exemple sur les fonctions acides en bout de chaîne d'un polymère pour donner des groupements esters. Ainsi le p-maléimidobenzoate de glycidyle de formule B ci-dessus est-il capable de réagir simultanément sur les deux composants d'un alliage polyéthylène-polybutylène téréphtalate (bouts de chaînes) pendant leur extrusion.

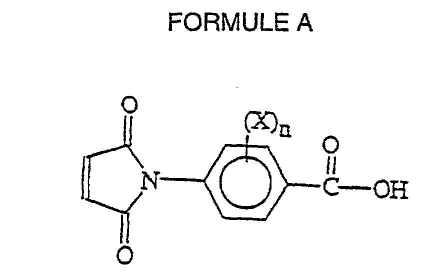

Les composés selon la présente invention de formule B ci-dessus peuvent être obtenus par réaction du chlorure de l'acide maléimidobenzoïque substitué ou non substitué sur le cycle benzénique sur un époxyalcool, par exemple le glycidol, au sein d'un solvant et en présence d'une base choisie dans le groupe formé par les amines tertiaires, telles que par exemple es trialcoylamines et en particulier la triéthylamine.

Le chlorure de l'acide maléimidobenzoïque substitué ou non substitué sur le cycle benzénique de formule F ci-après est un composé facilement obtenu, par exemple par réaction du chlorure de thionyle sur l'acide maléimidobenzoïque correspondant, en présence d'une base. En particulier, le chlorure de l'acide maléimidobenzoïque non substitué (composé de formule F dans laquelle n est égal à zéro) est décrit dans la littérature par exemple par B.S. RAO dans J. of Polym. Sci. (C) vol 26 pages 3 à 10, 1988.

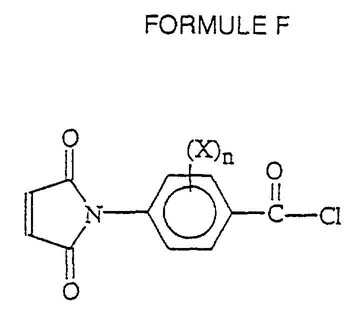

Les composés de formule B selon la présente invention peuvent être employés comme agents de fonctionnalisation des polyoléfines, en particulier des polyéthylènes, par greffage radicalaire de ces composés sur la chaîne paraffinique de la polyoléfine. Ce greffage peut être effectué en présence d'amorceur radicalaire ou en l'absence d'amorceur radicalaire. On préfère le plus souvent ne pas employer d'amorceur radicalaire, ce qui permet d'éviter tout risque de coupure et de réticulation de la polyoléfine. Ces composés sont également utilisables comme agents de compatibilisation de polymères incompatibles, ce qui permet en particulier de former des alliages de polymères. On pourra en particulier employer les composés de la présente invention pour former des alliages de polyoléfines avec des polybutylène-téréphtalates ou des polyamides.

L'exemple qui suit illustre l'invention sans en limiter la portée.

### Exemple

On mélange 71 g (0,33 M) d'acide maléimidobenzoïque de formule A dans laquelle n est égal à zéro, 750 ml de toluène et 0,7 g de pyridine et on coule rapidement 80 ml (1,08 M) de chlorure de thionyle. On chauffe en agitant à 70°C pendant deux heures et trente minutes. Après refroidissement, le solvant et l'excès de réactif sont éliminés sous vide. Le chlorure d'acide cristallise rapidement. Le solide obtenu a un point de fusion de 152,5 °C. Le point de fusion de ce corps donné dans l'article de F.J. LIU et Coll. publié dans J. of Polym. Sci. (A) vol. 30, pages 157 à 162, 1992, est de 153 °C. Le spectre infrarouge du produit au sein d'un pastille de KBr fait apparaître les bandes caractéristiques de la fonction imide et du chlorure d'acide : bande large à 1780 cm⁻¹ et 1720 cm⁻¹ (CO imide et chlorure d'acide) ; 1390 (imide); 1370, 1205, 1180, 1140 et 880 cm⁻¹ (chlorure d'acide).

On dissout dans 600 ml de dichloro-1,2 éthane, 35 (0,34 M) de triéthylamine et 25,5 g (0,34 M) de glycidol et on refroidit cette solution vers + 7°C par un bain froid à - 10°C. On ajoute alors 77 g (0,33 M) de chlorure d'acide obtenu comme décrit ci-dessus (de formule F dans laquelle n est égal à zéro), en 30 minutes environ avec un contrôle de température : minimum 7°C, maximum 12°C. Après addition, le mélange est agité à l'ambiante (environ 22°C) pendant 2 heures. La suspension est directement chromatographiée sur un fritté (diamètre 19 cm, hauteur 45 mm) rempli de 600 g de silicagel, par élution au moyen de dichloro-1,2 éthane (3,5 litres). Après évaporation du solvant, on isole 71,5 g d'un solide beige fondant à 90 °C (rendement : 80 %). L'ester de formule B, dans laquelle n est égal à zéro et R est un groupe méthylène, est recristallisé à partir du méthanol. Le point de fusion du solide recristallisé est de 93 °C. Le spectre de résonance magnétique nucléaire montre la présence d'acide maléimidobenzoïque (quadruplet des protons aromatiques à 8,05 et 7,5 ppm dans le DMSO deutéré). Cet acide de formule A dans laquelle n est égal à zéro provient, soit d'une hydrolyse pendant la purification par chromatographie sur silicagel (acide), soit d'une séparation imparfaite pendant la chromatographie. Le spectre infrarouge du produit au sein d'une pastille de KBr montre les bandes caractéristiques de la fonction ester et de la fonction imide à 1780 et 1720 cm⁻¹ (CO ester et imide) ; 1380 cm⁻¹ (imide) ; 1270 cm⁻¹ (ester).

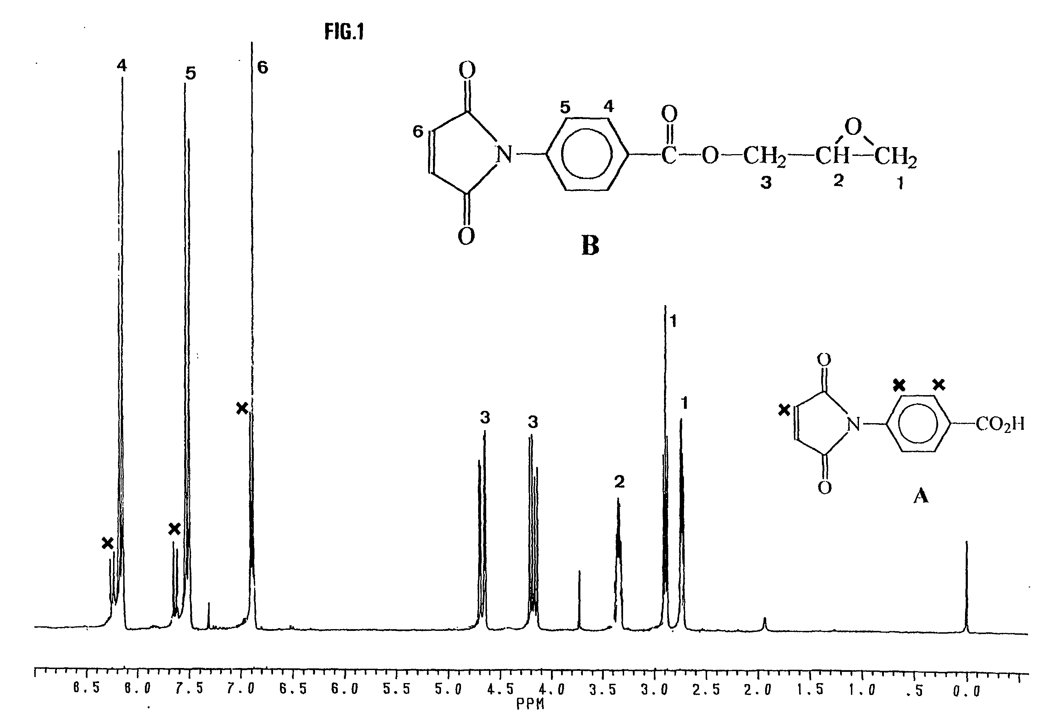

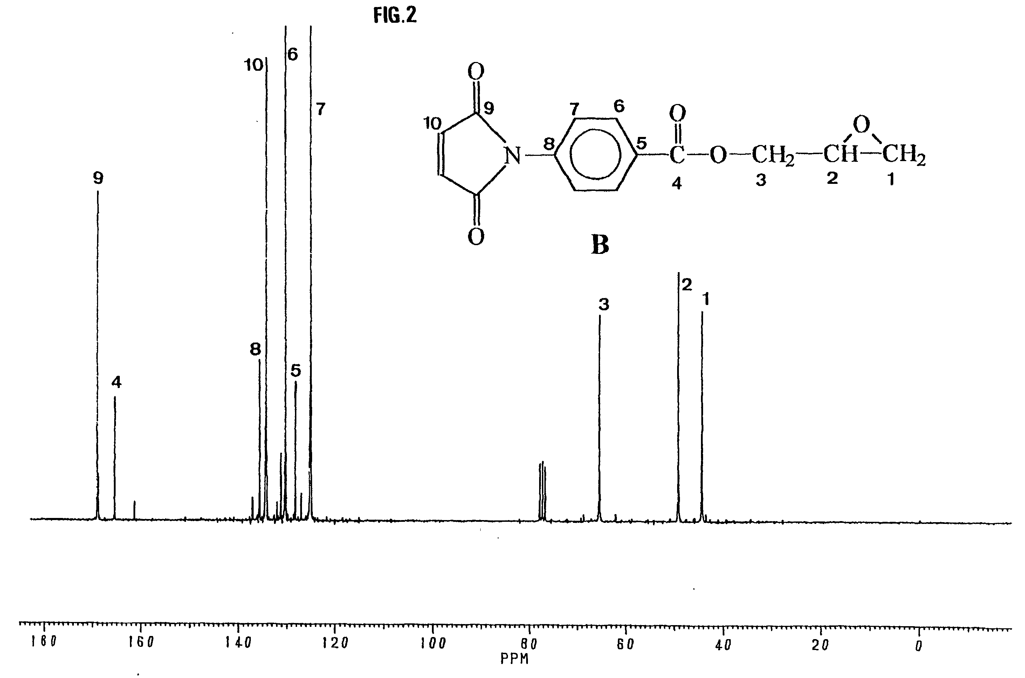

Les spectres RMN proton et carbone du produit obtenu sont illustrés par les figures 1 et 2.

## Revendications

1. Composés bifonctionnels **caractérisés en ce qu'**ils répondent à la formule générale : 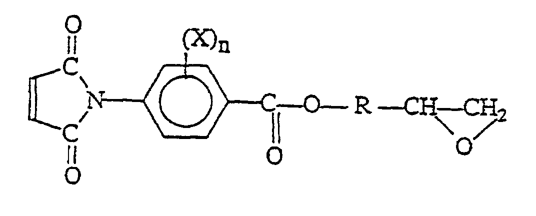 dans laquelle n est zéro ou un nombre entier de 1 à 4, bornes incluses, X est un un reste hydrocarboné ou un atome d'halogène, les groupes X étant identiques ou différents lorsque n est un nombre de 2 à 4, et R est un groupe divalent choisi parmi les groupes hydrocarbonés substitués ou non substitués.

2. Composé selon la revendication 1 dans lequel R est un groupe. hydrocarboné non substitué.

3. Composé selon l'une des revendications 1 et 2 dans lequel R est le groupe méthylène.

4. Composé selon l'une des revendications 1 à 3 dans lequel n est égal à zéro.

5. Procédé de préparation d'un composé selon l'une des revendications 1 à 4 dans lequel on fait réagir au sein d'un solvant un chlorure d'acide de formule: 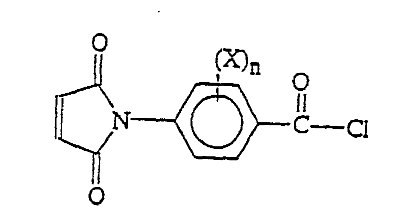 dans laquelle X et n ont la signification donnée dans la revendication 1, 2, 3 ou 4, sur un époxyalcool en présence d'une base choisie dans le groupe formé par les amines tertiaires.

6. Procédé de préparation selon la revendication 5 dans lequel la base est une trialcoylamine tertiaire.

7. Procédé de préparation selon la revendication 5 ou 6 dans lequel la base est la triéthylamine.

8. Utilisation d'un composé selon l'une des revendications 1 à 4 ou d'un composé préparé par un procédé selon l'une des revendications 5 à 7 comme agent de fonctionnalisation d'une polyoléfine par greffage de ce composé sur la chaîne paraffinique.

9. Utilisation d'un composé selon l'une des revendications 1 à 4 ou d'un composé préparé selon l'une des revendications 5 à 7 comme agent compatibilisant de polymères incompatibles.

10. Utilisation selon la revendication 9 pour la compatibilisation d'une polyoléfine avec un polybutylène-téréphtalate ou un polyamide.

## Patentansprüche

1. Bifunktionelle Verbindungen, **dadurch gekennzeichnet, dass** sie der allgemeinen Formel entsprechen: 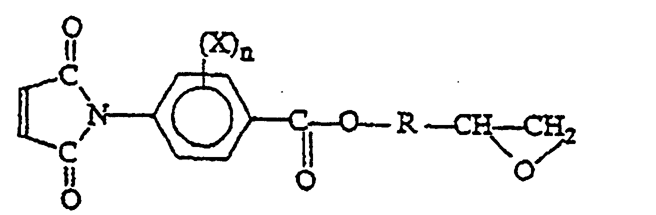 in der n Null oder eine ganze Zahl von 1 bis 4, Grenzwerte eingeschlossen, ist, X ein Kohlenwasserstoffrest oder ein Halogenatom ist, wobei die Gruppen X identisch oder verschieden sind, wenn n eine Zahl von 2 bis 4 und R eine divalente, unter den substituierten oder nicht substituierten Kohlenwasserstoffgruppen gewählte Gruppe ist.

2. Verbindung nach Anspruch 1, bei der R eine nicht substituierte Kohlenwasserstoffgruppe ist.

3. Verbindung nach einem der Ansprüche 1 und 2, bei der R die Methylengruppe ist.

4. Verbindung nach einem der Ansprüche 1 bis 3, bei der n gleich Null ist.

5. Verfahren zur Herstellung einer Verbindung gemäß den Ansprüchen 1 bis 4, bei dem man in einem Lösungsmittel ein Säurechlorid der Formel reagieren lässt: 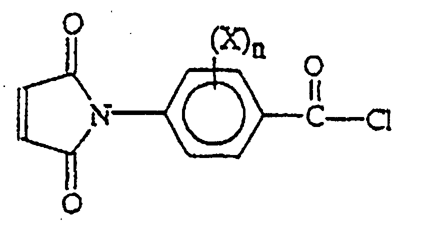 in der X und n die in den Ansprüchen 1, 2, 3 und 4 für einen Epoxyalkohol angegebene Bedeutung haben, in Gegenwart einer in der durch die tertiären Amine gebildeten Gruppe gewählten Base.

6. Verfahren zur Herstellung nach Anspruch 5, bei dem die Base ein tertiäres Trialcoylamin ist.

7. Verfahren zur Herstellung nach einem der Ansprüche 5 oder 6, bei dem die Base Triethylamin ist.

8. Verwendung einer Verbindung nach einem der Ansprüche 1 bis 4 oder nach einem Verfahren gemäß einem der Ansprüche 5 bis 7 hergestellten Verbindung als Funktionalisierungsreagenz eines Polyolefins durch Pfropfen dieser Verbindung auf die Paraffinkette.

9. Verwendung einer Verbindung nach einem der Ansprüche 1 bis 4 oder einer nach einem der Ansprüche 5 bis 7 hergestellten Verbindung als kompatiblisierendes Reagens für inkompatible Polymere.

10. Verwendung nach Anspruch 9 für die Kompatiblisierung eines Polyolefins mit einem Polybutylen-Terephtalat oder einem Polyamin.

## Claims

1. Bifunctional compounds **characterized in that** they have the following general formula: 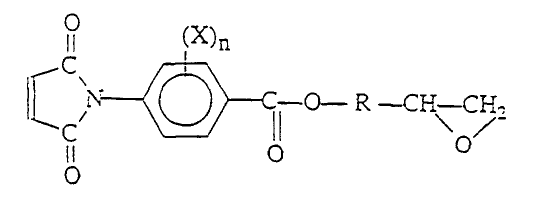 where n is zero or a whole number in the range 1 to 4, limits included, X is a hydrocarbon residue or a halogen atom, where the X groups are identical or different when n is a number from 2 to 4, and R is a divalent group selected from the substituted or unsubstituted hydrocarbon groups.

2. A compound according to claim 1, in which R is an unsubstituted hydrocarbon group.

3. A compound according to any one of claims 1 and 2, in which R is a methylene group.

4. A compound according to any one of claims 1 to 3, in which n equals zero.

5. A process for the preparation of a compound according to any one of claims 1 to 4, in which an acid chloride with the following formula: 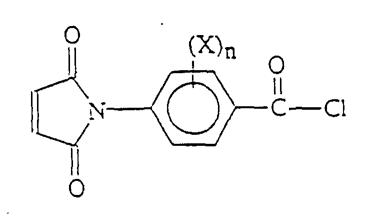 where X and n have the meanings given in claim 1, 2, 3 or 4 is reacted with an epoxyalcohol in a solvent in the presence of a relatively strong base selected from the group formed by tertiary amines.

6. A preparation process according to claim 5, in which the base is a tertiary trialkoylamine.

7. A preparation process according to claim 5 or claim 6, in which the base is triethylamine.

8. Use of a compound according to any one of claims 1 to 4 or of a compound prepared by a process according to any one of claims 5 to 7 as a functionalising agent for a polyolefin by grafting said compound onto the paraffinic chain.

9. Use of a compound according to any one of claims 1 to 4 or of a compound prepared according to any one of claims 5 to 7 as a compatibilising agent for incompatible polymers.

10. Use according to claim 9 for compatibilising a polyolefin with a polybutylene-terephthalate or a polyamide.
